# EUROPEAN PATENT APPLICATION

(11) **EP 0 956 904 A1**
(43) Date of publication of application: **17.11.1999**
(21) Application number: 99108628.1
(22) Date of filing: 11.05.1999
(51) Int. Cl.: B05B 1/02, B05B 11/04, B65D 47/18

(54) **Nozzle for dispensing liquids in drop form**

(30) Priority: 14.05.1998 IT MO980023
(71) Applicant: LAMEPLAST S.R.L., 41030 Rovereto di Novi (Modena) (IT)
(72) Inventor: Fabbri, Evro, 41030 Rovereto di Novi (Prov. of Modena) (IT); Ferrari, Giovanni, 41012 Carpi (Prov. of Modena) (IT); Fontana, Antonio, 41012 Carpi (Prov. of Modena) (IT)
(74) Representative: Modiano, Guido, Dr.-Ing.

(57) **Abstract**

A nozzle for dispensing liquids in drop form, comprising means (2) for hermetic coupling on the mouth of a bottle made of deformable material and an elongated dispensing duct (3) having a liquid inlet end (4) which has a diameter smaller than the diameter of said duct.

## Description

The present invention relates to a nozzle for dispensing liquids in drop form.

Bottles for liquids, such as medicines and the like, are conventionally made of a deformable material and have a mouth which is coupled hermetically to a nozzle which allows the liquid contained to flow out in the form of drops which have various weights and dimensions according to the outlet diameter of the nozzle.

These nozzles are not free from drawbacks, including the fact that they do not ensure effective control over the dimensions of the dispensed drop of liquid and do not allow the user to check the amount of liquid actually dispensed for each application, because said amount depends on the pressure applied to the deformable bottle.

Failure to control the outflow of the dispensed liquid also causes inevitable waste, with additional costs for the production of said liquid.

The aim of the present invention is to eliminate the above drawbacks of conventional nozzles by providing a nozzle for dispensing liquids in drop form which allows to perfectly control the dimensions of the drop of liquid being dispensed, to provide dispensing strictly in drop form regardless of the intensity of the pressure applied to the deformable bottle, to ensure effective control over the amount of liquid dispensed for each application, and to significantly reduce waste thereof.

Within the scope of this aim, an object of the present invention is to provide a nozzle for dispensing liquids in drop form having a structure which is simple, relatively easy to provide in practice, safe in use, effective in operation, and of relatively low cost.

This aim, this object and others which will become apparent hereinafter are achieved by the present nozzle for dispensing liquids in drop form, characterized in that it comprises means for hermetic coupling on the mouth of a bottle made of deformable material and an elongated dispensing duct having a liquid inlet end which has a diameter smaller than the diameter of the duct.

Further characteristics and advantages of the present invention will become apparent from the following detailed description of a preferred but not exclusive embodiment of a nozzle for dispensing liquids in drop form, according to the invention, illustrated only by way of non-limitative example in the accompanying drawings, wherein:
Figure 1 is a sectional view of a nozzle for dispensing liquids in drop form according to the invention;
Figure 2 is a sectional view of a second embodiment of the last portion of the duct of the nozzle according to the invention;
Figure 3 is a sectional front view of a third embodiment of the last portion of the duct of the nozzle according to the invention;
Figure 4 is a sectional front view of a fourth embodiment of the last portion of the duct of the nozzle according to the invention.

With particular reference to the above Figures, the reference numeral 1 generally designates a nozzle for dispensing liquids in drop form according to the invention.

The nozzle 1 is substantially ogive-shaped and has means 2 for hermetic coupling on the mouth of a bottle made of deformable material, not shown, and an elongated dispensing duct 3 whose liquid inlet end 4 has a very small diameter 5.

The duct 3 is coaxial to the nozzle 1, has a diameter which diverges toward the liquid outlet end 6, i.e. toward the end where the drop forms, and continues at the diameter 7 with a final portion 8.

The diameter 5 of the inlet end 4 is preferably between 0.1 and 0.3 millimeters, while the diverging diameter of the duct 3 forms an angle α which is between 0 and 30° and allows to form drops whose dimensions increase according to the breadth of said angle.

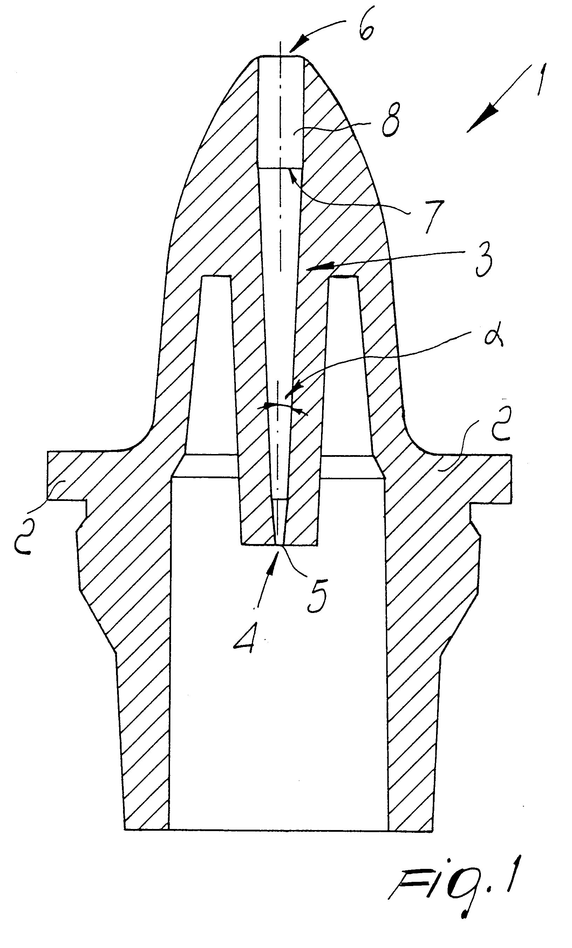

In Figures 1 and 3, the duct 3 has a last portion 8 with a constant diameter which is equal to, and greater, respectively, than diameter 7.

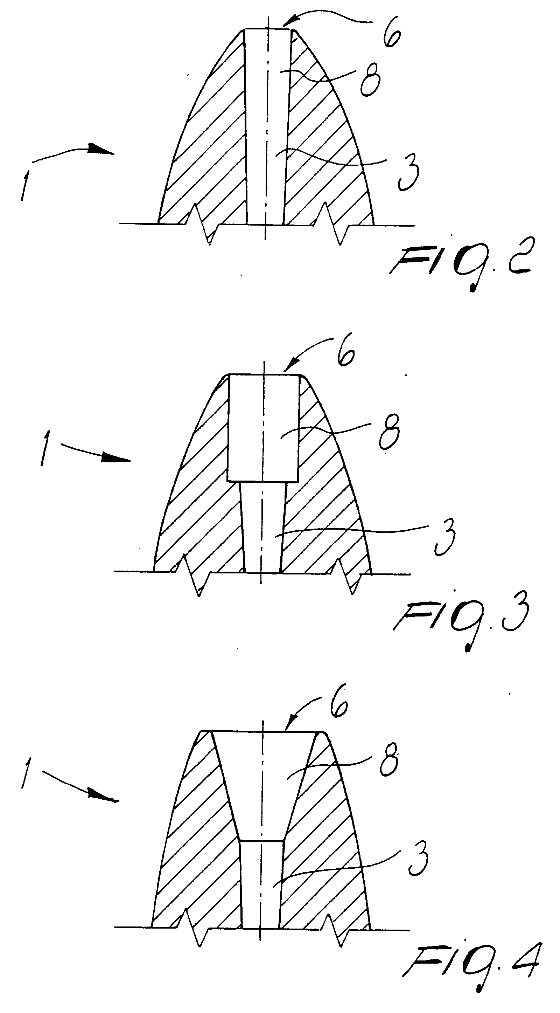

As an alternative, in the embodiments shown in Figures 2 and 4, the portion 8 of the duct 3 has a diameter which diverges toward the outlet end; moreover, in the example of Figure 4 the divergence of the portion 8 is greater than the divergence of the duct 3.

Once the diameter 5 has been set, different weights are obtained for the drop that forms according to the diameter 7, to the angle α and to the portion 8, which in turn determine the length of the duct 3.

For example, if the diameter 5 is equal to 0.2 millimeters, the diameter 7 is equal to 1.2 millimeters and the angle is equal to 2° 30', a drop weighing 0.030 milliliters forms.

Advantageously, there may be a different embodiment of the nozzle 1 in which the duct 3 has a constant diameter instead of diverging toward the outlet, as shown in the Figures.

It has thus been found that the invention achieves the intended aim and object, i.e. it allows to perfectly control the dimensions of the drop of liquid being dispensed according to the diameters of the inlet end and outlet end of the nozzle and according to the divergence angle.

The invention thus conceived is susceptible of numerous modifications and variations, all of which are within the scope of the inventive concept.

All the details may further be replaced with other technically equivalent ones.

In practice, the materials used, as well as the shapes and the dimensions, may be any according to requirements without thereby abandoning the scope of the protection of the appended claims.

The disclosures in Italian Utility Model No. MO98U000023 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A nozzle for dispensing liquids in drop form, characterized in that it comprises means for hermetic coupling on the mouth of a bottle made of deformable material and an elongated dispensing duct having a liquid inlet which has a diameter smaller than the diameter of said duct.

2. The nozzle according to claim 1, characterized in that said duct has a constant diameter.

3. The nozzle according to claim 1, characterized in that said duct has a diameter which diverges toward an outlet or drop formation end.

4. The nozzle according to claim 3, characterized in that a terminal portion of the outlet end of said duct has a constant diameter.

5. The nozzle according to claim 3, characterized in that a terminal portion of the outlet end of said duct has a diameter which diverges toward the outlet end.

6. The nozzle according to claim 5, characterized in that said portion diverges more than said duct.

7. The nozzle according to claim 1, characterized in that the diameter of said inlet end is between 0.1 and 0.3 millimeters.

8. The nozzle according to claim 3, characterized in that said diverging diameter forms an angle between 0 and 30° in order to form drops of increasing size.

9. The nozzle according to claim 1, characterized in that said duct is axial.

10. The nozzle according to claim 1, characterized in that it is ogive-shaped.
